# EUROPEAN PATENT APPLICATION

(11) **EP 2 301 418 A1**
(43) Date of publication of application: **30.03.2011**
(21) Application number: 10180908.5
(22) Date of filing: 28.09.2010
(51) Int. Cl.: A61B 1/04, A61B 1/07, A61B 5/00

(54) **Medical apparatus and endoscope apparatus**

(30) Priority: 29.09.2009 JP 2009225406; 25.12.2009 JP 2009296218; 29.06.2010 JP 2010148390
(71) Applicant: Fujifilm Corporation, Minato-ku Tokyo (JP)
(72) Inventor: Mizuyoshi, Akira, Ashigarakami-gun Kanagawa (JP); Kagaya, Hiroto, Ashigarakami-gun Kanagawa (JP); Torii, Yuichi, Ashigarakami-gun Kanagawa (JP); Iida, Takayuki, Ashigarakami-gun Kanagawa (JP)
(74) Representative: Klunker . Schmitt-Nilson . Hirsch

(57) **Abstract**

A medical apparatus includes an insertion unit and a light source which supplies light into the insertion unit. A surface of the insertion unit includes: a first and second irradiation portions, a second irradiation portions, and an observation window. wherein each of the first and second irradiation portions has a pair of irradiation windows which emits the light, wherein when a border line bisects a front end surface of the insertion unit through the center point of the observation window, the pair of the irradiation windows are disposed on the both side of the front end surface sandwiching the border line, and wherein a fluorescent body emitting light by the excitation of the light supplied from the light source unit is disposed behind each of optical paths, and the white light is formed by the light supplied to the first irradiation portions and emission in the fluorescent body.

## Description

### BACKGROUND OF INVENTION

### Field of the Invention

The present invention relates to a medical apparatus and an endoscope apparatus.

### Description of the Related Art

Generally, in an endoscope apparatus, an irradiation window and an observation window are disposed at the front end of an endoscope insertion unit, and an observation image is acquired through the observation window by emitting illumination light from the irradiation window. For example, light generated from a white light source such as a xenon lamp is guided to the irradiation window by a light guiding member such as an optical fiber bundle, and is emitted toward an observation area. Recently, a laser light source having a configuration in which white illumination light is formed by light obtained from the excitation of the fluorescent body has been used instead of the white light source.

In addition, particularly in the field of a medical endoscope, an endoscope apparatus capable of performing therapy or an observation using particular light (narrow bandwidth of light) having a specific narrow bandwidth of wavelength in addition to a general observation using the white illumination light also has been used. For example, JP-A-2009-34224 discloses an endoscope apparatus which includes a blue light emitting diode in addition to a lamp emitting light of an infrared light region or a visible light region. According to this kind of endoscope apparatus, it is possible to perform an observation in which capillaries or the like of a surface layer of a mucous membrane are emphasized, in addition to the general observation.

However, when the irradiation window emitting the illumination light from the endoscope front end portion is not disposed at an appropriate position with respect to the observation window used for obtaining the image information using an imaging element, the uniform illumination light cannot be emitted from the irradiation window. In the configuration of JP-A-2009-34224 below, since the irradiation window is disposed on the side of the observation window, the irregularity of the illumination easily occurs when there is an uneven portion in the observation area. In addition, since the positional relationship of the irradiation window in use is different for each of the general observation and the observation using particular light, it is not easy to make both illumination conditions equal to each other. Overlapping of pixel noises easily occurs when performing an image processing in accordance with a general observation image and a particular light observation image due to the difference in the illumination condition of the both observation images.

### SUMMARY OF INVENTION

An object of the invention is to provide a medical apparatus and an endoscope apparatus capable of performing various types of illumination on an observation area under satisfactory conditions at all times when a general observation using white light and an observation using particular light such as narrow bandwidth of light are performed.

The present invention has the following configuration.
(1) According to an aspect of the invention, medical apparatus includes:
   an insertion unit which inserts into a test object;
   a light source which supplies light into the insertion unit;
   wherein a surface of the insertion unit includes:
      a first irradiation portions which emits white light to the test object,
      a second irradiation portions which emits narrow bandwidth of light having short wavelength, which is shorter than the white light, and
      an observation window which observes the emitted test object, and
   wherein each of the first and second irradiation portions has a pair of irradiation windows which emits the light,
   wherein when a border line bisects a front end surface of the insertion unit through the center point of the observation window, the pair of the irradiation windows of the first irradiation portions are disposed on the both side of the front end surface sandwiching the border line and the pair of the irradiation windows of the second irradiation portions are disposed on the both side of the front end surface sandwiching the border line,
   wherein a fluorescent body emitting light by the excitation of the light supplied from the light source unit is disposed behind each of optical paths of the pair of the irradiation windows of the first irradiation portions, and the white light is formed by the light supplied to the first irradiation portions and emission in the fluorescent body.
(2) In the endoscope apparatus according to (1), the plurality of irradiation windows and the observation window are disposed on a front end of the insertion unit to be inserted into a body cavity, and the light sources supplies the light to at least one of the plurality of irradiation windows via at least one leading light units.

According to the medical apparatus and the endoscope apparatus, it is possible to perform each of the illumination under satisfactory conditions without causing irregularity of the illumination when the general observation using the white light and the observation using the particular light such as narrow bandwidth of light are performed. For example, it is possible to more appropriately perform a diagnosis or therapy using the endoscope.

### BRIEF DESCRIPTION OF THE DRAWINGS

Fig. 1 is a diagram illustrating an embodiment of the invention, and is a conceptual block diagram of an endoscope apparatus,
Fig. 2 is an external view of an example of the endoscope apparatus shown in Fig. 1,
Fig. 3A is a cross-sectional configuration diagram of a light projecting unit provided with a fluorescent body, and Fig. 3B is a cross-sectional configuration diagram of a light projecting unit provided with a light deflection/diffusion member,
Fig. 4 is a graph showing a light emission spectrum of blue laser light from a laser light source, and the result obtained by the wavelength conversion of the blue laser light through the fluorescent body,
Fig. 5 is a perspective view showing a schematic configuration of an endoscope front end unit,
Fig. 6 is an exploded view of the endoscope front end unit shown in Fig. 5,
Fig. 7 is a cross-sectional view taken along the line A-A of Fig. 5,
Fig. 8 is a front view when seen from the direction B of the endoscope front end unit shown in Fig. 5,
Fig. 9 is an explanatory diagram showing an illumination pattern from the light projecting unit,
Fig. 10 is a graph showing spectral characteristics of light absorption degrees of reduced hemoglobin and oxidized hemoglobin,
Figs. 11A to 11D are explanatory diagrams schematically showing the arrangement positions of the light projecting unit,
Fig. 12 is a front view when seen from the direction B of the endoscope front end unit of Fig. 5,
Fig. 13 is a plain view showing a front end surface of the endoscope front end unit,
Fig. 14 is a schematic cross sectional view showing the endoscope front end unit, and
Fig. 15 is a graph showing a detected light from an observation window per color.

### DESCRIPTION OF EXEMPLARY EMBODIMENTS

Hereinafter, an embodiment of the invention will be described with reference to the accompanying drawings.

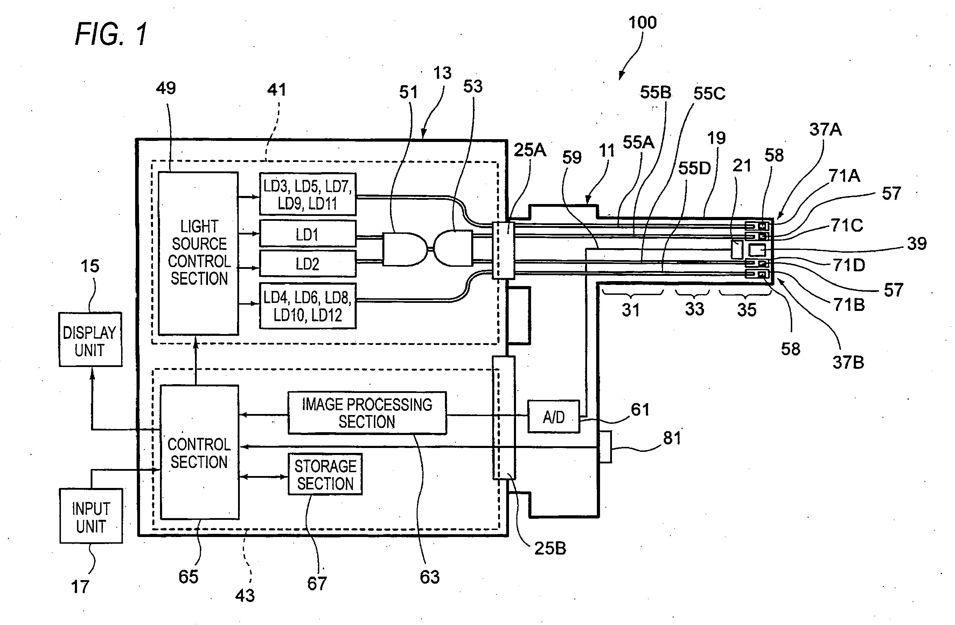

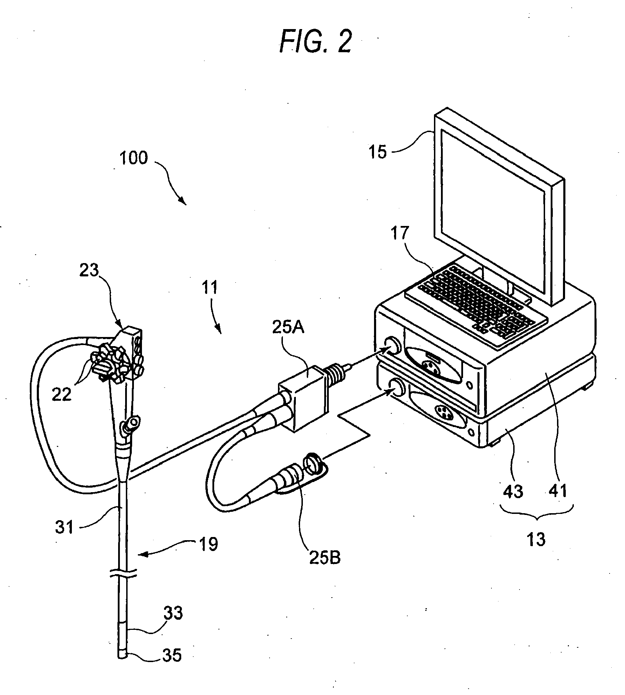

Fig. 1 is a diagram illustrating an embodiment of the invention, and is a conceptual block diagram of an endoscope apparatus. Fig. 2 is an external view of an example of the endoscope apparatus shown in Fig. 1.

As shown in Figs. 1 and 2, an endoscope apparatus 100 as one of medical apparatuses includes an endoscope 11, and a control device 13 to which the endoscope 11 is connected. The control device 13 is connected to a display unit 15 which displays image information or the like, and an input unit 17 which receives an input operation. The endoscope 11 is an electronic endoscope which includes an illumination optical system emitting an illumination light from a front end of an endoscope insertion unit 19 to be inserted into a test object and an imaging optical system including an imaging element 21 (refer to Fig. 1) configured to capture an image of an observation area.

In addition, the endoscope 11 includes the endoscope insertion unit 19, an operation unit 23 (refer to Fig. 2) which is used for an operation of curving the front end of the endoscope insertion unit 19 or an observation operation, and connectors 25A and 25B which are used to attachably/detachably connect the endoscope 11 to the control device 13. In addition, although not shown in the drawings, various channels such as a clamp channel used for inserting a tissue pickup treatment tool or the like therethrough or an air/water feeding channel are installed inside the operation unit 23 and the endoscope insertion unit 19.

The endoscope insertion unit 19 includes a flexible portion 31 which has flexibility, a curved portion 33, and a front end portion (hereinafter, referred to as an endoscope front end portion) 35. As shown in Fig. 1, the endoscope front end portion 35 is provided with illumination ports 37A and 37B which are used to emit light to the observation area, and an imaging sensor 21 such as a CCD (Charge Coupled Device) image sensor or a CMOS (Complementary Metal-Oxide Semiconductor) image sensor which is used to acquire image information of the observation area. In addition, an object lens unit 39 is disposed on the side of the light receiving surface of the imaging element 21.

The curved portion 33 is provided between the flexible portion 31 and the front end portion 35, and is operable to be curved by a rotation operation of an angle knob 22 disposed in the operation unit 23 shown in Fig. 2. The curved portion 33 is operable to be curved to an arbitrary direction and an arbitrary angle in accordance with a portion of the test object examined by the endoscope 11. The observation direction of the illumination ports 37A and 37B and the imaging element 21 of the endoscope front end portion 35 are operable to be directed to a desired observation portion. The structures of the illumination ports 37A and 37B of the endoscope insertion unit 19 will be described below in detail.

The control device 13 includes a light source device 41 which generates an illumination light to be supplied to the illumination ports 37A and 37B of the endoscope front end portion 35, and a processor 43 which performs an image process on an image signal generated from the imaging element 21, and is connected to the endoscope 11 via the connectors 25A and 25B. In addition, the processor 43 is connected to the display unit 15 and the input unit 17 which are described above. The processor 43 performs an image process on an imaging signal transmitted from the endoscope 11 on the basis of the command from the operation unit 23 or the input unit 17 of the endoscope 11, and generates a display image to be supplied to the display unit 15.

The light source device 41 includes a plurality of types of laser light sources having different central light emission wavelengths. In this configuration example, as shown in Fig. 1, a laser light source LD1 having a central light emission wavelength of 405 nm, a laser light source LD2 having a central light emission wavelength of 445 nm, and laser light sources LD3 and LD4 each having a central light emission wavelength of 405 nm are provided as a basic configuration. Further, in this configuration, laser light sources LD5 and LD6 each having a central light emission wavelength of 472 nm, laser light sources LD7 and LD8 each having a central light emission wavelength of 665 nm, laser light sources LD9 and LD10 each having a central light emission wavelength of 785 nm, and laser light sources LD11 and LD12 each having a central light emission wavelength of 375 nm are provided by commonly using the optical paths of the laser light sources LD3 and LD4. Each of the provided laser light sources may be in the range of plus or minus 10 nm on the central light emission wavelengths.

Each of the laser light sources LD 1 to LD12 is individually controlled by a light source control section 49 to emit light, and the laser lights may be generated individually or simultaneously. In addition, the light emission timing or the light amount ratio of each laser light source may be arbitrarily changed. Light spectrum from the irradiation windows which emits each laser light may be individually changed.

The laser light source LD1 is a narrow bandwidth of light observation light source which emits a violet laser light having a central wavelength of 405 nm, and the laser light source LD2 is a general observation light source which emits a blue laser light having a central wavelength of 405 nm so as to generate a white illumination light by providing a fluorescent body as a wavelength conversion member to be described later. In addition, the laser light sources LD3 and LD4 each generating a laser light having a central wavelength of 405 nm are fluorescent observation light sources, and are operable to emit light toward the observation area without using a fluorescent body to be described later.

The laser lights having a central wavelength of 472 nm and emitted from the laser light sources LD5 and LD6 are used to acquire information on a blood vessel depth and an oxygen saturation in blood. In addition, the laser lights having a central wavelength of 665 nm and emitted from the laser light sources LD7 and LD8 are therapeutic laser lights, and are used to perform photodynamic therapy (PDT) for curing a tumor such as cancer by illuminating a surface of a body tissue using a laser light having a comparatively strong output. Further, the laser lights having a central wavelength of 785 nm and emitted from the laser light sources LD9 and LD10 are used for the infrared light observation of ICG (Indocyanine Green) injected into a blood vessel. The laser light having a central wavelength of 375 nm emitted from the laser light sources LD11 and LD 12 becomes a light source for performing fluorescent observation by providing luciferase.

In addition, the laser light generated from the laser light source LD 1 is used as a laser light source for performing photodynamic diagnosis (PDD). The PDD is a diagnosis method which is conducted in such a manner that a photosensitive substance having tumor affinity and sensitive to a particular excitation light is injected into a body in advance, a laser light as an excitation light is made to illuminate the surface of the body tissue at a comparatively weak output, and then the fluorescence of the portion having increasing concentration of the light sensitive substance in diseased portions of tumor such as cancer is observed. The PDT therapy is performed on the diseased portions specified by the PDD.

As the laser light sources LD1 to LD12, an InGaN-based laser diode of a broad area type, and an InGaNAs-based laser diode or a GaNAs-based laser diode may be used. In addition, as the light source, a light emitting element such as a light emitting diode may be used. Further, in addition to the semiconductor light emitting element, a white light source such as a xenon lamp may be used to select the wavelength thereof by the use of a color filter.

The laser lights emitted from the laser light sources LD1 to LD12 are input to an optical fiber by a condenser lens (not shown). The laser lights emitted from the laser light sources LD1 and LD2 are multiplexed by a combiner 51 to be propagated via one way light optical path. Then, the multiplexed laser light is demultiplexed by a coupler 53, and is propagated to the connector 25A via two way light optical paths. Accordingly, the laser lights emitted from the laser light sources LD1 and LD2 are equally propagated to optical fibers 55A and 55C while speckles or differences of the light emission spectrums caused by the individual differences of the laser light sources are reduced. In addition, when the laser lights emitted from the laser light sources LD1 and LD2 are directly sent to the connector 25A without using the combiner 51 and the coupler 53, the configuration may be simplified.

The laser lights emitted from the laser light sources LD1 to LD12 are introduced into the optical fibers 55A to 55D which is extended from the connector 25A to the endoscope front end portion 35, at the arbitrary timings. The laser lights emitted from the laser light sources LD1 and LD2 are propagated to the fluorescent body 57 disposed at the endoscope front end portion 35. The laser lights emitted from the laser light sources LD3 to LD12 are propagated to a light diffusion member 58 and are emitted toward the observation area as illumination light (or therapeutic light).

Each of the optical fibers 55A to 55D is a multi-mode fiber. As an example, a thin fiber cable may be used which has a core diameter of 105 µm, a cladding diameter of 125 µm, and a diameter of φ0.3 to 0.5 mm including a protection layer as an outer surface.

Here, the optical fiber 55A and the fluorescent body 57 constitute a light projecting unit 71A, and the optical fiber 55B and the light diffusion member 58 constitute a light projecting unit 71B. In addition, the optical fiber 55C and the fluorescent body 57 constitute a light projecting unit 71D, and the optical fiber 55D and the light diffusion member 58 constitute a light projecting unit 71C. A pair of light projecting units 71A and 71D and a pair of light projecting units 71B and 71C are respectively disposed on both sides of the object lens unit 39 on the front end of the endoscope front end portion 35.

### [02[0002]

Next, the configuration of the light projecting units of the endoscope front end portion will be described.

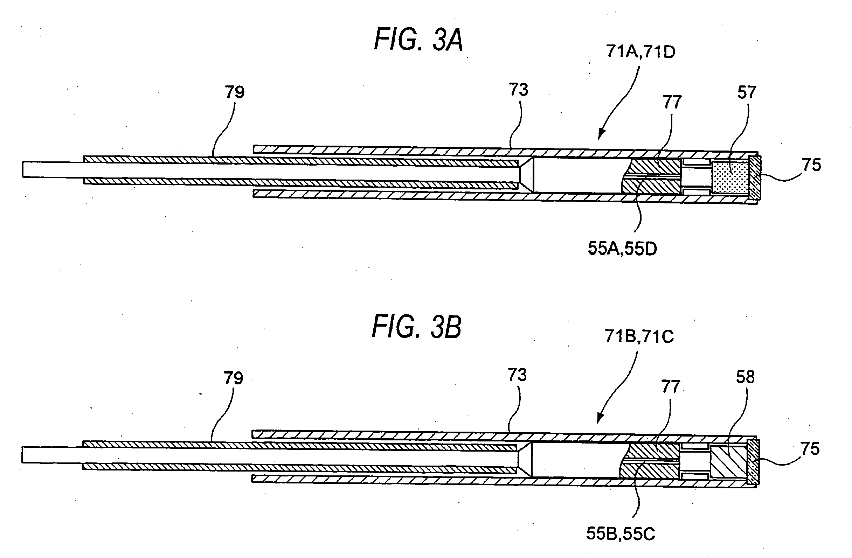

Fig. 3A is a cross-sectional configuration diagram of the light projecting units 71A and 71D, and Fig. 3B is a cross-sectional configuration diagram of the light projecting units 71B and 71C. The light projecting unit 71A and the light projecting unit 71D have the same configuration, and each includes the fluorescent body 57, a cylindrical sleeve member 73 which covers the outer periphery of the fluorescent body 57, a protective glass (irradiation window) 75 which seals one end side of the sleeve member 73, and a ferrule 77 which is inserted into the sleeve member 73 to support the optical fiber 55A (55C) along the central axis. In addition, in the optical fiber 55A (55C) extending from the rear end side of the ferrule 77 while being covered by an outer surface, a flexible sleeve 79 covering the outside of the outer surface is inserted between the outer surface and the sleeve member 73.

On the other hand, the light projecting unit 71B and the light projecting unit 71C have the same configuration, and have the same configuration as that of the light projecting units 71A and 71D except that the light deflection/diffusion member 58 is disposed instead of the fluorescent body 57 of the light projecting units 71A and 71D, and the light is guided from the optical fibers 55B and 55D.

The fluorescent body 57 of each of the light projecting units 71A and 71 D includes a plurality of types of fluorescent substances (for example, a YAG-based fluorescent body or a fluorescent body such as BAM (BaMgAl₁₀O₁₇))which absorbs a part of the blue laser light emitted from the laser light source LD2 and is excited to emit light of green to yellow. Accordingly, white (color similar to white) illumination light is formed as the result of synthesizing green to yellow excitation light emission lights using blue laser light as excitation light with the blue laser light not absorbed and transmitted through the fluorescent body 57.

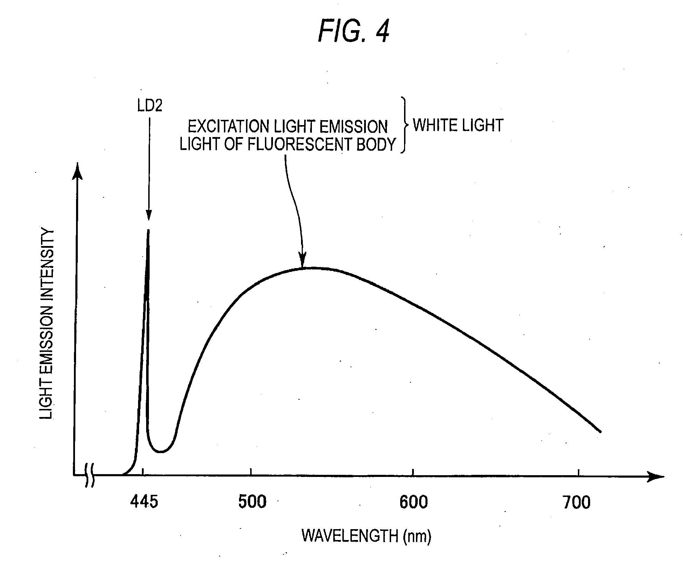

Fig. 4 is a graph showing a light emission spectrum of the blue laser light emitted from the laser light source LD2, and the result obtained by the wavelength conversion of the blue laser light through the fluorescent body 57. The blue laser light is depicted by the bright line having a central wavelength of 445 nm, and the excitation light emission light emitted from the fluorescent body 57 as a result of a reaction with the blue laser light has a spectral intensity distribution in which the light emission intensity substantially increases in the bandwidth of the wavelength of 450 nm to 700 nm. The above-described white light is formed by the profile of the excitation light emission light and the blue laser light. According to this configuration example, when the semiconductor light emitting element is used as the excitation light source, it is possible to obtain white light having high intensity and high light emission efficiency. Also, it is possible to easily adjust the intensity of the white light, and to minimally suppress variations in chromaticity and tone of the white light.

Here, the white light mentioned in the specification precisely includes not only all wavelength components of the visible light, but also for example, reference colors of R (Red), G (Green), B (Blue), and the like of the light of the specific wavelength. For example, the light including the wavelength component from green to red or the light including the wavelength component from blue to green is included in a broad sense in the white light.

The fluorescent body 57 may prevent an occurrence of flickering when performing a video display or overlapping of noise as a barrier in the image capturing operation due to a speckle generated by coherence of laser light. In addition, in the fluorescent body 57, in consideration of a difference in the refractive index between the fluorescent substance forming the fluorescent body and a fixation/solidification resin as a filling agent, it is desirable that the particles of the filling agents and the fluorescent substance are formed of a material having large scattering and small absorption with respect to the infrared light. Accordingly, it is possible to improve the scattering effect without reducing the light intensity with respect to the light of red or infrared region.

In addition, the light deflection/diffusion members 58 of the light projecting units 71B and 71C may be formed of a material which transmits the laser lights emitted from the laser light sources LD3 to LD12, and for example, a translucent resin material having translucent ceramic, glass, and the like are used. Further, the light diffusion member 58 may have a configuration in which the surface or the intermediate layer of the resin material or glass is provided with a light diffusion layer having minute uneven portions thereon or particles (fillers and the like) having mixed different refractive indexes, or may be formed by the use of a translucent material. Accordingly, the transmitted light emitted from the light diffusion member 58 becomes narrow bandwidth of light having a uniform light amount within a predetermined illumination area due to deflection operation and diffusion operation of the light.

Returning to Fig. 1, the description is continued. As described above, the white light formed by the blue laser light and the excitation light emission light generated from the fluorescent body 57, and the narrow bandwidth of lights generated by the laser lights are emitted from the front end portion 35 of the endoscope 11 toward the observation area of the test object. In addition, the shape of the observation area illuminated by the illumination light is photographed by the imaging element 21 by forming the image of the test object using the object lens unit 39.

The imaging signal of the captured image output from the imaging element 21 after capturing image is transmitted to an A/D converter 61 via a scope cable 59 to be converted into a digital signal, and the digital signal is input to an image processing section 63 of the processor 43 via the connector 25B. The image processing section 63 performs various processes such as white balance correction, gamma correction, contour emphasis, and color correction on the captured image signal output from the imaging element 21 and converted into the digital signal. The captured image signal processed by the image processing section 63 is formed as an endoscope observation image together with a variety of information by a control section 65, and the result is displayed on the display unit 15. In addition, if necessary, the result is stored in a storage section 67 configured as a memory or a storage device.

Next, one configuration example of the endoscope front end portion will be described in detail.

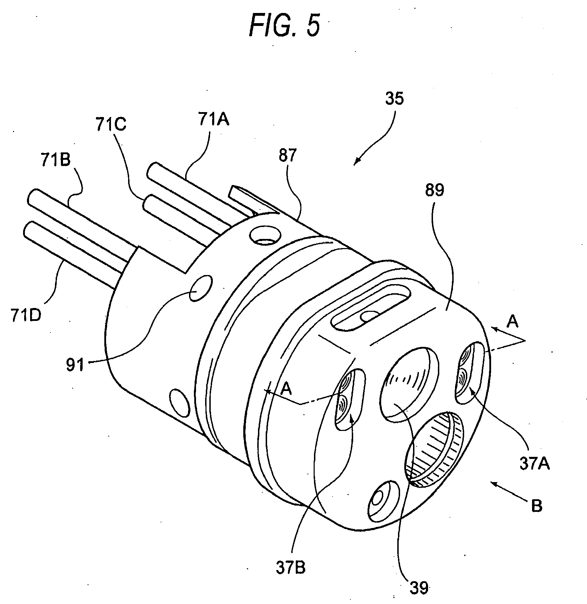

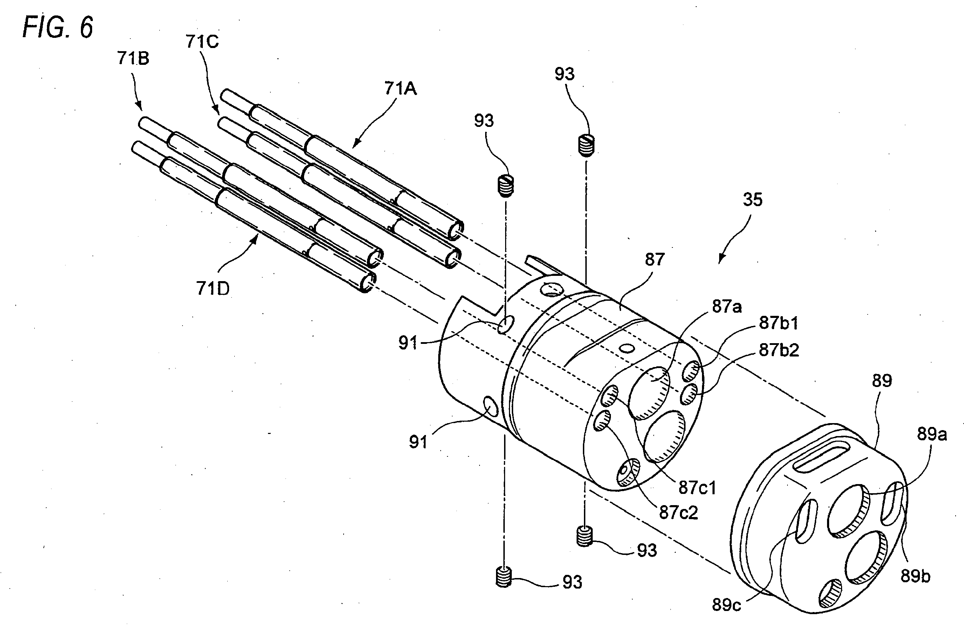

Fig. 5 is a perspective view showing a schematic configuration of the endoscope front end portion, and Fig. 6 is an exploded diagram of the endoscope front end portion shown in Fig. 5.

As shown in Figs. 5 and 6, in the endoscope front end portion 35, various components such as the light projecting units 71A to 71D are mounted to a front end rigid portion 87 which is formed of stainless steel or the like and has a plurality of perforation holes formed along the longitudinal direction. The front end rigid portion 87 includes a perforation hole 87a which accommodates the imaging optical system including the imaging element 21 shown in Fig. 1, and perforation holes 87b1, 87b2, 87c1, and 87c2 are formed on both sides of the perforation hole 87a. The light projecting units 71A and 71C are respectively inserted into the perforation holes 87b1 and 87b2, and the light projecting units 71B and 71D are respectively inserted into the perforation holes 87c1 and 87c2.

In addition, the front end side of the front end rigid portion 87 is covered by a front end rubber cap 89, and the outer periphery of the front end rigid portion 87 is covered by an outer surface tube (not shown). The front end rubber cap 89 is provided with perforation holes 89a, 89b, 89c, and the like which respectively correspond to the perforation holes 87a, 87b1, 87b2, 87c1, 87c2, and the like of the front end rigid portion 87, and also the observation window of the object lens unit 39 or the illumination ports 37A and 37B of the light projecting units 71A to 71D are opened therefrom.

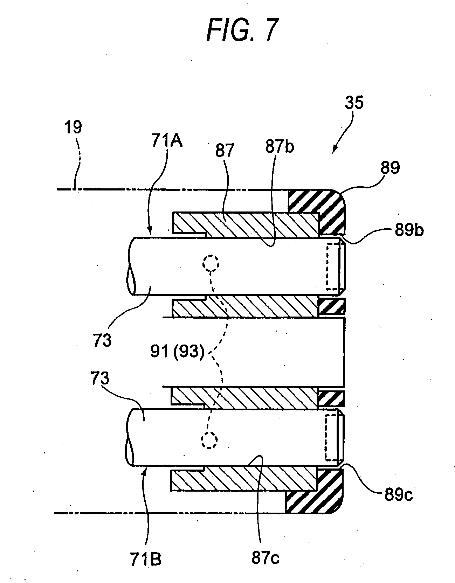

Here, the cross-sectional view taken along the line A-A of Fig. 5 is shown in Fig. 7A. The light projecting units 71A and 71B are fixed to the front end rigid portion 87 in such a manner that the light projecting units 71A and 71B are inserted into the perforation holes 87b1 and 87c1 of the front end rigid portion 87, and a locking screw 93 is fastened from a pair of horizontal holes 91 (refer to Figs. 4 and 5) communicating with the perforation holes 87b1 and 87c1. In addition, the light projecting units 71C and 71D are also fixed to the front end rigid portion 87 by fastening the locking screw 93 in this manner.

According to the configuration of the endoscope with the above-described light projecting units 71A to 71D, since the light projecting units 71A to 71D are attachably/detachably fixed by the locking screw 93 while being press-inserted into the perforation holes 87b1, 87b2, 87c1, and 87c2 of the front end rigid portion 87, it is possible to easily exchange the light projecting units 71A to 71D, and to improve the facilitation of the maintenance for the endoscope. That is, when there are phenomena such as a variation in the tone or attenuation of the intensity of the illumination light due to a long period of usage of the endoscope, it is possible to easily exchange the current light projecting unit with a new one.

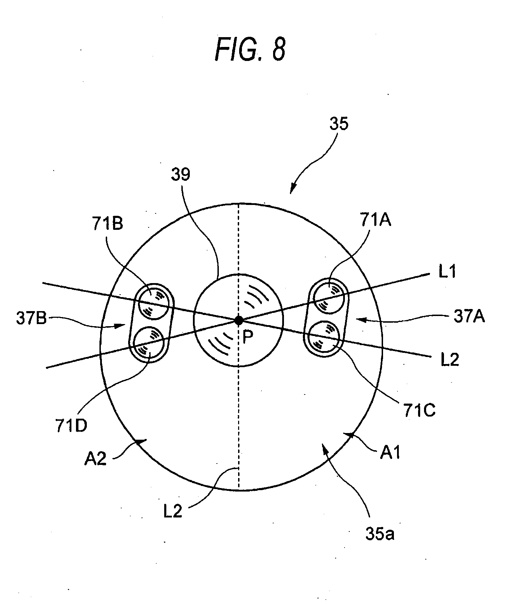

Fig. 8 is a front view when seen in the direction B of the endoscope front end portion shown in Fig. 5. As described above, the light projecting units 71A and 71C and the light projecting units 71B and 71D are disposed on both sides of the object lens unit 39 so that the light projecting units 71A and 71C emit light from the illumination port 37A, and the light projecting units 71B and 71D emit light from the illumination port 37B. In addition, the pair of light projecting units 71 A and 71D each having the fluorescent body (refer to Fig. 3A) is disposed so that the line L1 connecting the positions of the protective glasses 75 (refer to Fig. 3) as the irradiation windows cross the lens area of the object lens unit 39 as the observation window. Further, the pair of light projecting units 71B and 71C each having the light diffusion member 58 (refer to Fig. 3B) is disposed so that the line L2 connecting the positions of the protective glasses 75 (refer to Fig. 3) crosses the lens area of the object lens unit 39.

The plurality of irradiation windows include a first light irradiation portions (the light projecting units 71A and 71D) formed by a pair of irradiation windows emitting the white light via the fluorescent body 57 and a second irradiation portions (the light projecting units 71B and 71 C) formed by a pair of irradiation windows emitting the narrow bandwidth of light having a narrower lightwave range than the white light's. When a border line L3 bisects a front end surface 35a of the endoscope front end portion 35 through the center point P of the observation window, the pair of the irradiation windows of the first irradiation portions are disposed on the both side of the front end surface 35a sandwiching the border line L3 and the pair of the irradiation windows of the second irradiation portions are disposed on the both side of the front end surface 35a sandwiching the border line L3.

That is, the first irradiation portions (the light projecting units 71A and 71D) of the irradiation windows and the second irradiation portions (the light projecting unit 71B and 71C) of the irradiation windows are disposed one by one in each front end surface range A1 and A2 bisected by the border line L3, respectively.

In this configuration example, the light projecting units 71A to 71D are disposed while improving the efficiency of the space so that the lines L1 and L2 have an intersection point P within the lens area of the object lens unit 39. That is, the light projecting units 71A and 71D emitting the white illumination light are disposed on both sides with the object lens unit 39 of the endoscope front end portion 35 interposed therebetween, and the white illumination lights are equally emitted from both sides of the object lens unit 39, thereby preventing the occurrence of the irregularity of the illumination.

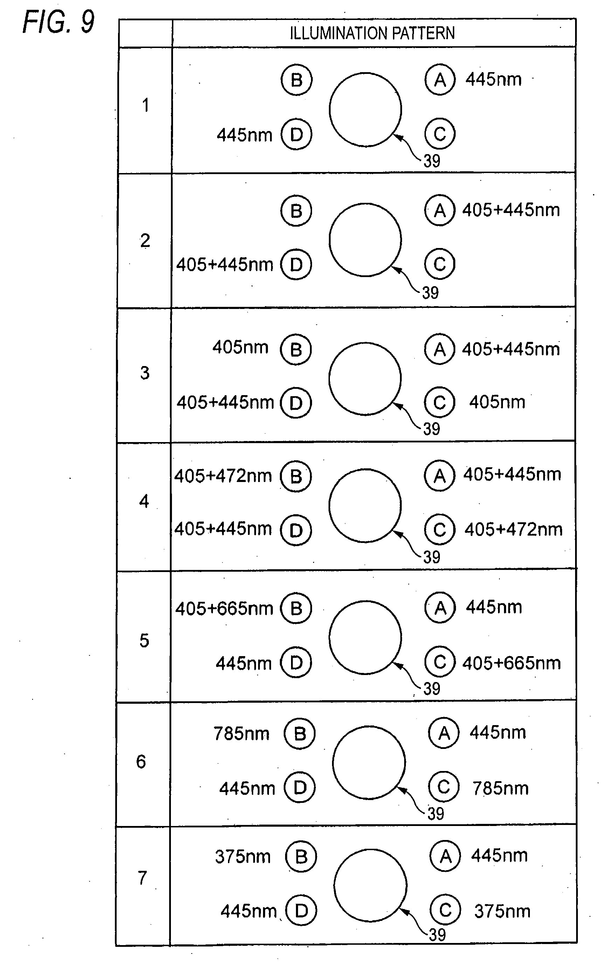

Next, each of the illumination patterns will be described, in which the laser lights generated from the laser light sources LD1 to LD12 are emitted through the light projecting units 71A to 71D by an appropriate combination thereof to thereby form various illumination lights. An example of each of the illumination patterns is shown in Fig. 9. The symbols A, B, C, and D on both sides of the object lens unit 39 in Fig. 9 respectively denote the light projecting units 71A, 71B, 71C, and 71D.

### <First illumination pattern>

The laser light having a central wavelength of 445 nm is introduced from the laser light source LD2 to the light projecting units 71A and 71D, and the white light is emitted from each of the light projecting units 71A and 71D. In addition, the light emission of the light projecting units 71B and 71C is stopped by turning off the outputs of the laser light sources LD3 to LD12.

This illumination pattern is used as an illumination pattern during general observation. Since the white lights are emitted from both the light projecting units 71A and 71D, the occurrence of shade is suppressed even when there is a protrusion portion in the observation area, thereby performing the general observation at the minimum irregular illumination.

### <Second illumination pattern>

The laser light (narrow bandwidth of light) having a central wavelength of 405 nm generated from the laser light source LD1 and the laser light (light forming the white light) having a central wavelength of 445 nm generated from the laser light source LD2 are respectively introduced to the light projecting units 71A and 71D. In addition, the light emission of the light projecting units 71B and 71C is stopped by turning off the outputs of the laser light sources LD3 to LD12. The illumination timings of the laser light sources LD1 and LD2 are appropriately controlled so that the illumination operations using the white light and the narrow bandwidth of light are simultaneously performed, in addition to the individual illumination operation using each of the white light and the narrow bandwidth of light having a short wavelength (violet).

According to this illumination pattern, since the narrow bandwidth of light is used for the illumination in addition to the white light, it is possible to perform observation in which capillaries of a surface layer of a mucous membrane are emphasized in addition to the general observation of the white illumination. In addition, by increasing the light emission amount of the laser light source LD1 more than that of the laser light source LD2, it is possible to observe a more detailed image on a surface layer of a mucous membrane in a near view. On the other hand, by increasing the light emission amount of the laser light source LD1 more than that of the laser light source LD2, it is possible to observe a brighter image in a distant view including wide information of the surface layer of the mucous membrane. Further, since the light emission amount ratio between the laser light sources LD1 and LD2 is arbitrarily changed, it is possible to observe a distribution of blood vessels of a surface layer in the depth direction.

The change of the light emission amount ratio between the laser light sources LD1 and LD2 may be performed at an arbitrary timing or a programmed specific timing by operating a conversion switch 81, the input unit 17, or the light source device 41 of the endoscope 11 shown in Fig. 1. In addition, when a predetermined light emission amount ratio is made to be selected in accordance with the operation of the switch, it is possible to simply switch the general observation image and the capillary emphasis image.

As described above, the lights emitted from the light projecting units 71A and 71D may be arbitrarily set to be supplied from any one of the laser light sources LD1 and LD2, and may be changed in accordance with an observation scene. For this reason, it is possible to freely extract necessary information in accordance with the observation scene, and to easily obtain an observation image appropriate for an observation purpose.

In addition, when the laser lights are simultaneously emitted from the same laser light sources LD1 and LD2, since the laser lights may be emitted from the light projecting units 71A and 71D in the same condition, both illumination conditions may be made to be equal with high precision when performing a calculation process on the observation image. As a result, it is possible to accurately extract a variation in the observation image due to a difference in the illumination light.

### <Third illumination pattern>

As in the second illumination pattern, the laser lights are introduced from the laser light sources LD1 and LD2 to the light projecting units 71A and 71D in accordance with an arbitrary light emission amount ratio. In addition, the laser lights having a central wavelength of 405 nm are introduced from the laser light sources LD3 and LD4 to the light projecting units 71B and 71C. The illumination of the pair of light projecting units 71A and 71D and the pair of light projecting units 71B and 71C is alternately performed every imaging frame, but is not performed simultaneously. That is, the image is captured by repeating a first frame in which the image is captured by the use of the narrow bandwidth of light having a short wavelength (violet) and the white light emitted from the light projecting units 71 A and 71D and a second frame in which the image is captured by the use of the excitation lights emitted from the light projecting units 71B and 71C. Then, the image of each frame is displayed on the display unit 15 (refer to Fig. 1). Alternatively, the images of the respective frames are synthesized and displayed.

According to this illumination pattern, since the narrow bandwidth of light having a short wavelength and the white light are emitted from the light projecting units 71A and 71D in accordance with an arbitrary light emission amount ratio, it is possible to obtain the capillary emphasis image in addition to the general observation image. In addition, since the excitation lights having a central wavelength of 405 nm are emitted from the light projecting units 71B and 71C, it is possible to obtain an observation image of self fluorescence from a fluorescent substance such as collagen present in a body, or an observation image for PDD. In Table 1, the wavelengths of the PDD excitation light, the PDD fluorescence, and the PDT therapeutic light are shown for each medicine, and the laser light having a central wavelength of 350 nm to 450 nm (the central wavelength of 405 nm may be highly preferable) may be used as the PDD excitation light even when any one of fluorescent medicines of photofrin, laserphyrin, visudyne, and 5-ALA (Amino Levulinic Acid) is used. In addition, due to the accumulation of protoporphyrin IX, the wavelength ratio of the fluorescence of the 5-ALA is changed in accordance with progression of the disease.

**[Table 1]**

| MEDICINE NAME | PDD EXCITATION LIGHT | PDD fluorescence | PDT therapeutic LIGHT |
|---|---|---|---|
| PHOTOFRIN | 405 nm | 660 nm | 630 nm |
| LASERPHYRIN | 405 nm | 660 nm | 664 nm |
| Visudyne | 405 nm | 660 nm | 689 nm |
| 5-ALA | 405 nm | 635/670 nm | 630 nm |

In addition, in the narrow bandwidth of lights having a short wavelength (violet) emitted from the light projecting units 71B and 71C, the laser lights emitted from the laser light sources LD3 and LD4 are emitted through the light diffusion member 58 (refer to Fig. 3B), but are not transmitted through the fluorescent body. For this reason, the light emission component of the fluorescent body does not appear as noise in the observation image. In addition, the light emission intensity is not reduced by the light absorption or the light diffusion of the fluorescent body. Further, since the same narrow bandwidth of lights are emitted from the plurality of light projecting units 71B and 71C, it is possible to obtain the illumination light which is distributed uniformly, and thus to improve the light intensity.

Further, the light to be emitted may be changed every frame, but also may be changed at an arbitrary timing or a programmed specific timing by operating the conversion switch 81, the input unit 17, or the light source device 41 of the endoscope 11 shown in Fig. 1.

### <Fourth illumination pattern>

At least one of the laser lights generated from the laser light sources LD1 and LD2 are introduced to the light projecting units 71A and 71D. On the other hand, at least one of the laser lights having a central wavelength of 405 nm generated from the laser light sources LD3 and LD4 and the laser lights having a central wavelength of 472 nm generated from the laser light sources LD5 and LD6 are introduced to the light projecting units 71B and 71C.

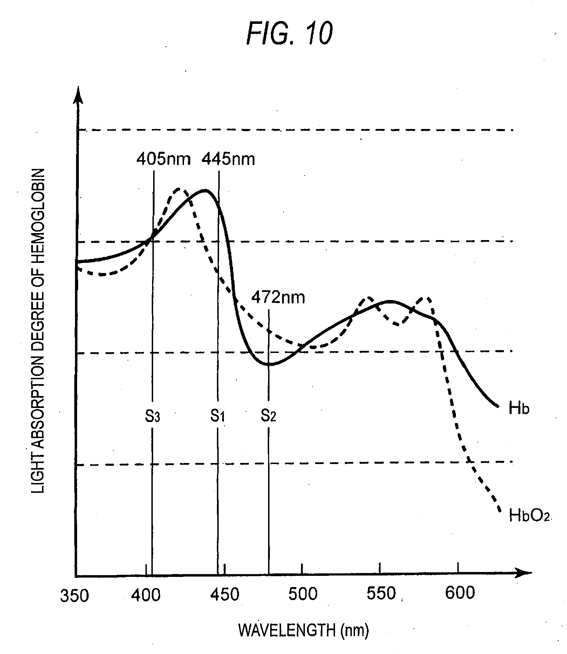

According to this illumination pattern, the oxygen saturation and the blood vessel depth of the observation area may be obtained by the use of a difference in the light absorption spectrum between reduced hemoglobin Hb obtained after oxygen discharge and oxidized hemoglobin HbO₂ in hemoglobins contained in red corpuscles in blood. In the oxidized hemoglobin HbO₂ and the reduced hemoglobin Hb, as shown in the spectral characteristics of the light absorption degree of Fig. 10, the light absorption degrees are substantially equal to each other around the wavelength of 405 nm, the light absorption degree of the reduced hemoglobin Hb is higher than that of the oxidized hemoglobin HbO₂ around the wavelength of 445 nm, and the light absorption degree of the oxidized hemoglobin HbO₂ is higher than that of the reduced hemoglobin Hb around the wavelength of 472 nm. In addition, the invasion depth of the laser light from the surface layer of the mucous membrane becomes shallower as the wavelength of the laser light becomes shorter.

By using such characteristics, for example, the oxygen saturation of the observation area and the blood vessel depth displayed in the observation area are obtained as below.
(1) A captured image luminance value S1 is obtained by detecting a returning light component of the laser light when performing the illumination by the use of the laser light having a central wavelength of 445 nm at which the light absorption degree of the reduced hemoglobin Hb is high.
(2) A captured image luminance value S2 is obtained by detecting a returning light component of the laser light when performing the illumination by the use of the laser light having a central wavelength of 472 nm at which the light absorption degree of the oxidized hemoglobin HbO₂ is high.
(3) A captured image luminance value S3 is obtained by detecting a returning light component of the laser light when performing the illumination by the use of the laser light having a central wavelength of 405 nm at which the light absorption degrees of the oxidized hemoglobin HbO₂ and the reduced hemoglobin Hb are substantially equal to each other.
(4) The values S1 and S2 are respectively normalized by the value S3. That is, the values of S1/S3 and S2/S3 are obtained.
(5) A two-dimensional map representing the magnitudes of the value S1/S3 and the value S2/S3 in the orthogonal two axes is created, and the value S1/S3 and the value S2/S3 are plotted on the two-dimensional map. In the two-dimensional map, as the value S1/S3 becomes larger, the oxygen saturation becomes higher and the blood vessel depth becomes shallower. On the other hand, as the value S1/S3 becomes smaller, the oxygen saturation becomes lower and the blood vessel depth becomes deeper. Further, as the value S2/S3 becomes larger, the oxygen saturation becomes lower and the blood vessel depth becomes shallower. On the other hand, as the value S2/S3 becomes smaller, the oxygen saturation becomes higher and the blood vessel depth becomes deeper. By the use of such relationships, the information on the saturation degree and the blood vessel depth in the observation area may be obtained.

The captured image luminance values S1, S2, and S3 are obtained from the captured image data which is acquired by alternating the illumination lights of the light projecting units. That is, at the time when the value S1 is obtained, the laser light having a central wavelength of 445 nm is introduced from the laser light source LD2 to the light projecting units 71A and 71D. In addition, the light emission of the light projecting units 71B and 71C is stopped by turning off the outputs of the laser light sources LD3 to LD12. In this case, fluorescence may be generated from the fluorescent body 57, but this fluorescence is generated at the long wavelength which is separated from the wavelength of the laser light emitted from the laser light source LD2. For this reason, since only the B imaging signal of the color imaging elements of R, G, and B is used, it is possible to detect only the returning light component of the laser light generated from the laser light source LD2 by selectively removing the fluorescence component.

At the time when the value S2 is obtained, the laser light having a central wavelength of 472 nm is introduced from the laser light source LD2 to the light projecting units 71B and 71C. In addition, the light emission of the light projecting units 71A and 71D is stopped by turning off the outputs of the laser light sources LD1 and LD2.

At the time when the value S3 is obtained, the laser light having a central wavelength of 405 nm is introduced from the laser light source LD1 to the light projecting units 71A and 71D. In addition, the light emission of the light projecting units 71B and 71C is stopped by turning off the outputs of the laser light sources LD3 to LD12. Even in this case, it is possible to selectively remove the fluorescence component generated from the fluorescent body 57 which is excited by the laser light of the laser light source LD1.

Alternatively, when the laser light having a central wavelength of 405 nm is introduced from the laser light sources LD3 and LD4 to the light projecting units 71B and 71C, and the light emission of the light projecting units 71A and 71D is stopped by turning off the outputs of the laser light sources LD1 and LD2, fluorescence is not generated from the fluorescent body 57.

Further, there is no effect from the fluorescent body 57 so as to set the laser light source (not shown) having a central wavelength of 445 nm being independent from the light source LD2 and introduce the laser light source having 445 nm into the projecting units 71B and 71C.

### <Fifth illumination pattern>

The blue laser light is introduced from the laser light source LD2 to the light projecting units 71 A and 71D to thereby form the white light. In addition, the laser light having a central wavelength of 405 nm generated from the laser light sources LD3 and LD4 and the laser light having a central wavelength of 665 nm generated from the laser light sources LD7 and LD8 are selectively introduced to the light projecting units 71B and 71C. The laser light having a central wavelength of 405 nm is used to obtain the observation image for PDD, and the laser light having a central wavelength of 665 nm is used as the therapeutic light for PDT.

As shown in Table 1, the wavelength of the therapeutic light for PDT is appropriately selected in accordance with the type of the medicine to be used, and generally, the laser light having a wavelength of 620 to 680 nm may be used.

According to this illumination pattern, it is possible to emit the PDD light beam and the PDT light beam from the light projecting units 71B and 71C in addition to the white light emitted from the light projecting units 71A and 71D. For example, firstly the PDD light beam is emitd in the body cavity after the endoscope front end portion is moved to the vicinity of the diseased portion inside the body cavity through the general observation using the white light. Then, the position of the diseased portion is specified by detecting a generated fluorescent portion due to the irradiation of the PDD light beam. Finally, the diseased portion is cured by emitting the PDT light beam towar the specified diseased portion. According to these procedures, at the time when the PDT is performed after the PDD, the endoscope does not need to be temporarily extracted from the body cavity, and the PDD and the PDT may be continuously performed while the endoscope is inserted into the body cavity. In addition, the PDT light beam may be emitted to the diseased portion in such a manner that a PDT light beam emission probe is inserted from a clamp hole of the endoscope, and the endoscope front end portion is protruded therefrom.

### <Sixth illumination pattern>

The blue laser light is introduced from the laser light source LD2 to the light projecting units 71 A and 71 D to thereby form the white light. In addition, the laser light of a near infrared region having a central wavelength of 785 nm is introduced from the laser light sources LD9 and LD10 to the light projecting units 71B and 71C. The laser light having a central wavelength of 785 nm is appropriately used to observe information on blood vessels of an in-depth layer of a mucous membrane, and blood vessel navigation or infrared light observation using ICG may be performed. This ICG is linked to a protein in blood. For example, the ICG absorbs a near infrared light having a wavelength of 750 to 850 nm (the maximum absorption wavelength is 805 nm), and generates a near infrared fluorescence.

According to this illumination pattern, since it is possible to emit the near infrared light in addition to the white light, particularly, the information on blood vessels of an in-depth layer of a mucous membrane, which is not easily obtained in the visible light, may be extracted. For example, in addition that the illumination pattern is applied to an endoscope navigation system used for obtaining positional information of blood vessels around a bronchial tube, the laser light having a central wavelength of 785 nm is emitted toward the ICG injected into the blood vessels. Then, since fluorescence having broad spectral characteristics with a peak wavelength of 830 nm is generated in the portion where a reaction between the ICG and blood occurs, it is possible to perform an accurate treatment with high positional precision by setting the generated fluorescence as a target. In addition, since a plurality of light projecting units is used, it is possible to perform the illumination by the use of the light having high intensity and obtained by a combination of the lights emitted from the light projecting units.

### <Seventh illumination pattern>

The white light is generated by introducing the blue laser beam from the laser light source LD2 to the projecting units 71A and 71D. In addition, the laser beam of the near infrared light having the central wavelength of 375 nm is introduced from the laser light sources LD11 and LD12 to the projecting units 71B and 71C.

Luciferase is exited by near ultraviolet light having the wavelength of 375 nm. Then, Luciferase generates fluorescence having the wavelength of 490 as maximum. Since invasion depth of the near ultraviolet light to a body tissue is shallow, according to the illumination pattern, it is possible to obtain the information of a surface layer of the mucous membrane which it is difficult to obtain by ICG. Further, it is possible to observe with high visibility since the fluorescence is generated in blue-green spectrum light.

According to each irradiation pattern, apart from the above-described configuration, any one of the laser lights emitted from the laser light sources LD5 to LD12 may be introduced to the fluorescent body 57 (refer to Fig. 3A). In this case, the fluorescent body 57 is formed of a material having a low absorption rate with respect to the wavelengths of the laser lights emitted from the laser light sources LD5 to LD12. Accordingly, since the excitation light emission amount is small even when the fluorescent body 57 is illuminated by the light, the observation image is not influenced by the mixed color of the white light.

Further, in any illumination pattern described above, the case of not simultaneously performing the illumination by alternately performing the illumination of the pair of light projecting units 71A and 71D and the pair of light projecting units 71B and 71C every imaging frame, and the case of simultaneously performing the illumination may be selectively set. In addition, when the illumination is alternately performed every frame, various display methods may be selectively set such that the captured frame image is displayed on the display unit 15 (refer to Fig. 1) every frame or the frame images are synthesized and displayed on the display unit 15.

Furthermore, in the above-described configuration, a pair of laser light sources LD1 and LD2 respectively having central wavelengths of 405 nm and 445 nm is disposed on the light source device 41, and the lights emitted from the laser light sources LD1 and LD2 are multiplexed. However, a configuration may be adopted in which a pair of laser light sources LD1 and LD2 is further additionally provided, and the lights emitted from the plurality of laser light sources LD1 and LD2 are multiplexed by a coupler or the like. In this case, the speckle or irregularity of the light emission wavelength due to the individual differences of the laser light sources is reduced, and the light emission wavelength of the fluorescent body may be set to the stipulated wavelength. In addition, one pair of the plurality of laser light sources LD1 and LD2 may be used as an auxiliary light source for the other pair to guarantee its reliability.

As described above, since the white light and the narrow bandwidth of light are used for the illumination by a combination of the plurality of light projecting units, it is possible to perform the general observation using the white light and the observation using particular lights such as the observation using the narrow bandwidth of light, the observation using the fluorescence, and the observation using the infrared light in the satisfactory illumination condition. In addition, since the narrow bandwidth of light is not transmitted through the fluorescent body for forming the white light, it is possible to perform the high-intensity illumination by the use of the narrow bandwidth of light without generating needless light components.

The endoscope apparatus 100 of this configuration is not limited to the above-described embodiment. For example, modifications and applications conducted by the person skilled in the art on the basis of the description of the specification and the generally known technologies may occur in the invention, and are included in the scope of claims required to be protected.

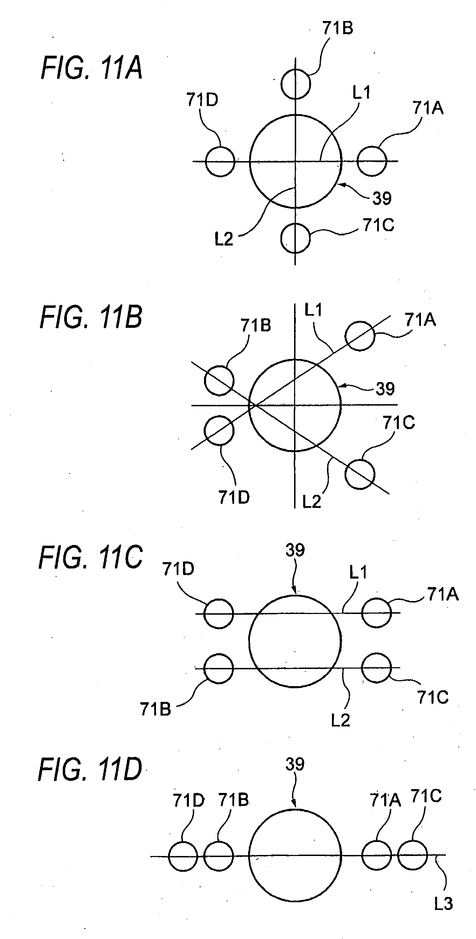

For example, as shown in Fig. 11A, the arrangement positions of the light projecting units may be set so that the line L1 connecting the light projecting units 71A and 71D relative to the object lens unit 39 as the observation window is perpendicular to the line L2 connecting the light projecting units 71B and 71C.

In addition, the light projecting units 71A to 71D may have an asymmetrical arrangement relationship as shown in Fig. 11B, and may have an arrangement relationship in which the lines L1 and L2 are parallel to each other without intersecting as shown in Fig. 11C. Further, the light projecting units 71A to 71D may be respectively disposed on the line L3 as shown in Fig. 11D.

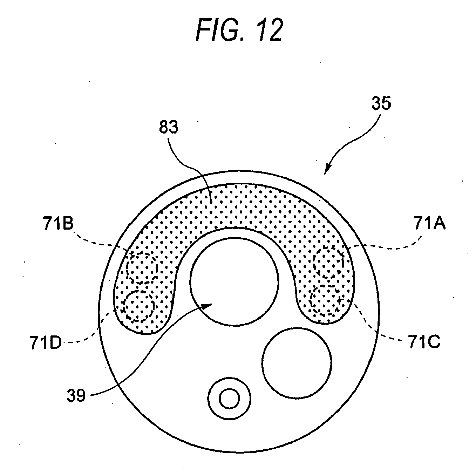

Further, the light may be emitted from the substantially entire part of a light diffusion plate 83 by disposing the light diffusion plate 83 at the light emission ends of the light projecting units 71A to 71D as shown in the front view of Fig. 12, which is a view when the endoscope front end portion is seen from the direction B of Fig. 5. In this case, even when the light having high intensity is emitted from the light projecting unit, the light is diffused by the light diffusion plate 83, and the illumination may be performed over the wide area of the light diffusion plate 83.

That is, the light having high density is not emitted from the narrow areas of the light emission ends of the light projecting units, and the uniform illumination light may be generated from the wide light emission surface, thereby further reducing the irregularity of the illumination.

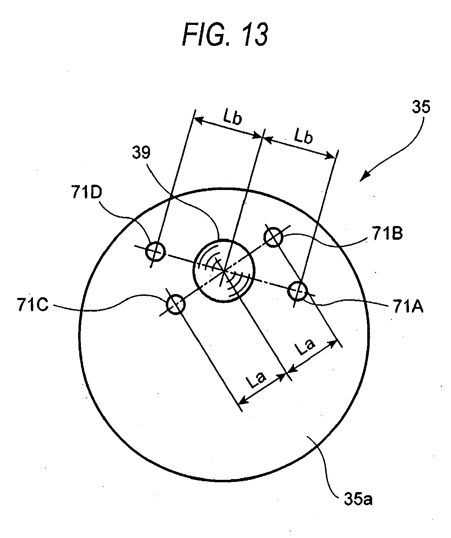

The irradiation windows emitting the illumination light of short wavelength may be disposed on closer to the observation window than the other irradiation window. Thus, the irregularity of light intensity which is different in each observation wavelength generating in near distance imaging is reduced. Fig. 13 shows the front end surface 35a of the endoscope front end portion 35. The two pairs of the irradiation windows are disposed on the front end surface 35a to cross the object lens unit 39 as a center thereof. The two pairs of the irradiation windows are the pair of projecting units 71B and 71C emitting the light with the short wavelength and the pair of projecting units 71A and 71D emitting the white light.

The projecting unit 71B and the projecting unit 71C are disposed at a position of distance La from the center of the observation window of the object lens unit 39, respectively. The projecting unit 71A and the projecting unit 71D are disposed at a position of distance Lb, which is longer than distance La, from the center of the observation window. Therefore, the projecting units 71B and 71C are disposed on inside of the projecting unit 71 A and 71D on the front end surface 35a.

As the above configuration, when the pair of first irradiation windows and the pair of second irradiation windows which emits the light of short wavelength, which is shorter than the pair of the first irradiation windows, are disposed on the front end surface 35a of the endoscope front end portion 35, disposing the pair of second irradiation windows inside the pair of first irradiation windows has advantages as explained below.

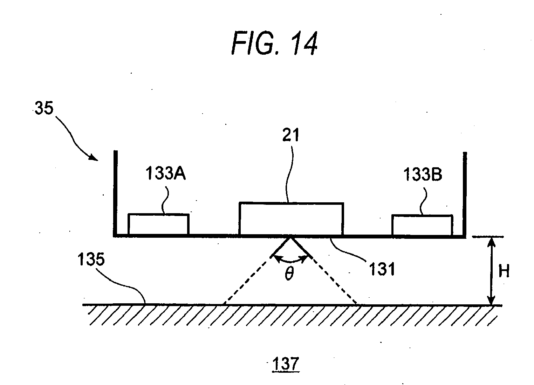

While angle of view of a general endoscope becomes about 120 to 140 degrees, angle of view in enlarged observation that the distance between the tip of the endoscope and an object becomes about 1 to 3 mm becomes about 120 to 140 degrees. Fig. 14 shows a schematic cross sectional view of the endoscope front end unit 35. When the imaging near distance such as distance H between the observation window 131 with the imaging element 21 and observation region 135 is performed, irregularity of the light intensity occurs to the observation image because the light reaching the center portion in the view is relatively less than the light reaching peripheral portion in the view.

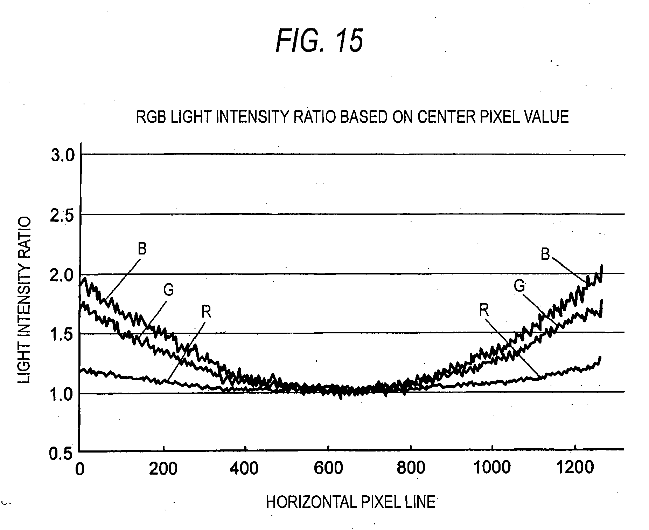

This irregularity of the light intensity is different in per color due to the following reason. Red color element R of light is relatively long wavelength in the light of observation windows 133A and 133B which are disposed on the same distance from the observation window 131 and which sandwiches the observation window 131. The red color element R of the light not only reflects from the surface of the observation region 135 of the body tissue but also is scattered inside of the body 137. As a result, although the scattering light reaches the central portion of the observation view, blue color element B of the short wavelength shortly decreases inside the body 137. Only the reflected light from the surface of the observation region 135 reaches the central portion of the observation view. Thus, the detected light from observation window 131 per color is shown as Fig. 15. Fig. 15 shows Light intensity ratio distribution of the detected light R, G and B based on center pixel value of the observation view. The irregularity of the light intensity of the red color element R of the light becomes small so that the red color element R of the light shows flat near the light intensity ratio of 1.0 in any position on the horizontal pixel line on the observation view. On the other hand, the light intensity ratio of green color element G and blue color element B of the light whose wavelength are shorter than the red color element R of the light becomes large at near positions to the irradiation windows 133A and 133B, that is peripheral region of the observation view, in comparison with the center of the horizontal pixel line. In the element of light having short wave length, the light intensity ratio becomes large at the peripheral region and the irregularity of the light intensity increase.

Therefore, when the irradiation window emitting the short wavelength light comes close to the observation window, it is possible to make it difficult to receive the effect of the irregularity of the light intensity by preventing the occurrence of shading in the near distance imaging.

In addition, the application of the above-described light projecting units 71A to 71D is not limited to the endoscope apparatus, but the light projecting units may be applied to different types of medical apparatuses such as a rigid scope, a scope endoscope, various surgical apparatuses, and a capsular electronic endoscope.

As described above, the present specification discloses the following items.
(1) According to an aspect of the invention, medical apparatus includes:
   an insertion unit which inserts into a test object;
   a light source which supplies light into the insertion unit;
   wherein a surface of the insertion unit includes:
      a first irradiation portions which emits white light to the test object,
      a second irradiation portions which emits narrow bandwidth of light having short wavelength, which is shorter than the white light, and
      an observation window which observes the emitted test object, and
   wherein each of the first and second irradiation portions has a pair of irradiation windows which emits the light,
   wherein when a border line bisects a front end surface of the insertion unit through the center point of the observation window, the pair of the irradiation windows of the first irradiation portions are disposed on the both side of the front end surface sandwiching the border line and the pair of the irradiation windows of the second irradiation portions are disposed on the both side of the front end surface sandwiching the border line,
   wherein a fluorescent body emitting light by the excitation of the light supplied from the light source unit is disposed behind each of optical paths of the pair of the irradiation windows of the first irradiation portions, and the white light is formed by the light supplied to the first irradiation portions and emission in the fluorescent body.
   According to the medical apparatus, since the pair of first irradiation windows and the pair of second irradiation windows are respectively disposed with the observation window interposed therebetween, it is possible to form the uniform illumination light in which the irregularity of the illumination does not occur. In addition, the light emitted from the light source unit and subjected to wavelength conversion and the light transmitted through the fluorescent body without wavelength conversion are emitted from the first irradiation windows, and the illumination light having a desired wavelength may be obtained. Accordingly, for example, it is possible to perform both the general observation using the white light and the observation using particular light having a specific wavelength in a satisfactory illumination condition.
(2) The medical apparatus according to claim 1, wherein the pair of irradiation windows of the first irradiation portions are disposed on both side of the observation window, and
   wherein the pair of irradiation windows of the second irradiation portions are disposed on both side of the observation window at a position which is different from the first irradiation portions.
   According to the medical apparatus, since the lights may be selectively emitted from the first and second irradiation windows by individually controlling the first and second light sources, it is possible to supply necessary light at an arbitrary timing.
(3) In the endoscope apparatus according to (1) or (2), the pair of irradiation windows of the second irradiation portions are disposed on more inside than the first irradiation portions.
   According to the medical apparatus, since the semiconductor light emitting element such as an LED light emitting element or a laser light emitting element having high intensity and high light emission efficiency is used, it is possible to reliably obtain the light having a uniform wavelength.
(4) In the endoscope apparatus according to (1) to (3), the irradiation windows disposed on the front end surface of the insertion unit so that a line linking the pair of irradiation windows of the first irradiation portions and a line linking the pair of irradiation windows of the second irradiation portions cross on the observation window, respectively.
   According to the medical apparatus, the lights emitted from the second irradiation windows are not subjected to wavelength conversion, but are diffused to perform the uniform illumination.
(5) The endoscope apparatus according to (1) to (4) may further include:
   a light diffusion member disposed behind optical paths of the second irradiation portions of the pair of irradiation windows.
   According to the medical apparatus, the plurality of lights having different wavelengths may be emitted from each of the first and second irradiation windows.
(6) In the endoscope apparatus according to (1) to (5), the light source unit includes a first light source supplying light to the first irradiation portions and a second light source supplying light to the second irradiation portions, and
   wherein the light source unit further includes a light source control unit individually controls light emission from the first light source and the second light source.
   According to the medical apparatus, the image of the test object illuminated by the lights emitted from the first and second irradiation windows may be acquired as image data, and various information processes such as an image calculation may be performed by using the image data.
(7) In the endoscope apparatus according to (6), the second light source has a light emitting element which generates narrow bandwidth of light of a plurality of different kinds of spectrum each other, and emits the light of the plurality of different kinds of spectrum each other from the second irradiation portion.
   According to the medical apparatus, it is possible to dispose a plurality of irradiation windows by improving the efficiency of the space.
(8) In the endoscope apparatus according to (7), the second light source generates that the central wavelength of the narrow bandwidth of light is 350 to 450 nm.
   According to the endoscope apparatus, since the lights are emitted from the first and second irradiation windows disposed with the observation window interposed therebetween, it is possible to illuminate the test object by the use of the uniform illumination light in which the irregularity of the illumination does not occur. Accordingly, it is possible to improve the precisions of examination and diagnosis using the endoscope.
(9) In the endoscope apparatus according to (11), the second irradiation windows generates (i) narrow bandwidth of light having a wavelength in which a light absorption degree of reduced hemoglobin in blood is substantially equal to a light absorption degree of oxidized hemoglobin and (ii) narrow bandwidth of light having a wavelength in which both of the light absorption degrees have difference.
   According to the endoscope apparatus, it is possible to perform the general observation using the illumination of the white light, and to perform a desired observation using the illumination of the narrow bandwidth of light.
(10) In the endoscope apparatus according to (1) to (9), the second light source generates that the central wavelength of the narrow bandwidth of light is 620 to 680 nm.
   According to the endoscope apparatus, since the narrow bandwidth of light emitted from the second irradiation windows is the narrow bandwidth of light having a central wavelength of 450 nm or less, for example, it is possible to perform an observation in which capillaries of a surface layer of a mucous membrane are emphasized by using, for example, narrow bandwidth of light having a wavelength of 405 nm or so. In addition, the narrow bandwidth of light may be used as diagnosis light for PDD. Further, an observation using self fluorescence of a body tissue may be performed.
(11) In the endoscope apparatus according to (1) to (10), the second light source generates that the central wavelength of the narrow bandwidth of light is 750 to 850 nm.
   According to the endoscope apparatus, since a plurality of types of lights having different wavelengths may be selectively or simultaneously emitted from the second irradiation windows, it is possible to select the appropriate illumination light in accordance with the purpose.
(12) In the endoscope apparatus according to (1) to (10), at least one the first and second light sources is made of semiconductor emitting element.
   According to the endoscope apparatus, it is possible to obtain the information on the oxygen saturation and the blood vessel depth of the test object by the use of the image calculation.
(13) The endoscope apparatus according to (1) to (12) may further include:
   an imaging element which detects image of the test object to be focused through the observation window and outputs observation view signal of the test object.
   According to the endoscope apparatus, the light emitted from the second irradiation windows may be used as the therapeutic light for PDT.
(14) In the endoscope apparatus according to (1) to (13), the plurality of irradiation windows and the observation window are disposed on a front end of the insertion unit to be inserted into a body cavity, and
   the light sources supplies the light to at least one of the plurality of irradiation windows via at least one leading light units.

According to the endoscope apparatus, the lights emitted from the second irradiation windows may be used as the excitation light for fluorescent medicine such as Indocyanine Green, and the observation using the fluorescence of the medicine may be performed.

## Claims

1. A medical apparatus comprising:
an insertion unit which inserts into a test object;
a light source which supplies light into the insertion unit;
wherein a surface of the insertion unit includes:
a first irradiation portions which emits white light to the test object,
a second irradiation portions which emits narrow bandwidth of light having short wavelength, which is shorter than the white light, and
an observation window which observes the emitted test object, and
wherein each of the first and second irradiation portions has a pair of irradiation windows which emits the light,
wherein when a border line bisects a front end surface of the insertion unit through the center point of the observation window, the pair of the irradiation windows of the first irradiation portions are disposed on the both side of the front end surface sandwiching the border line and the pair of the irradiation windows of the second irradiation portions are disposed on the both side of the front end surface sandwiching the border line,
wherein a fluorescent body emitting light by the excitation of the light supplied from the light source unit is disposed behind each of optical paths of the pair of the irradiation windows of the first irradiation portions, and the white light is formed by the light supplied to the first irradiation portions and emission in the fluorescent body.

2. The medical apparatus according to claim 1, wherein the pair of irradiation windows of the first irradiation portions are disposed on both side of the observation window, and
wherein the pair of irradiation windows of the second irradiation portions are disposed on both side of the observation window at a position which is different from the first irradiation portions.

3. The medical apparatus according to claim 1 or 2, wherein the pair of irradiation windows of the second irradiation portions are disposed on more inside than the first irradiation portions.

4. The medical apparatus according to any one of claims 1 to 3, wherein the irradiation windows disposed on the front end surface of the insertion unit so that a line linking the pair of irradiation windows of the first irradiation portions and a line linking the pair of irradiation windows of the second irradiation portions cross on the observation window, respectively.

5. The medical apparatus according to any one of claims 1 to 4, further comprising:
a light diffusion member disposed behind optical paths of the second irradiation portions of the pair of irradiation windows.

6. The medical apparatus according to any one of claims 1 to 5,
wherein the light source unit includes a first light source supplying light to the first irradiation portions and a second light source supplying light to the second irradiation portions, and
wherein the light source unit further includes a light source control unit individually controls light emission from the first light source and the second light source.

7. The endoscope apparatus according to claim 6, wherein the second light source has a light emitting element which generates narrow bandwidth of light of a plurality of different kinds of spectrum each other, and emits the light of the plurality of different kinds of spectrum each other from the second irradiation portion.

8. The endoscope apparatus according to claim 7, wherein the second light source generates that the central wavelength of the narrow bandwidth of light is 350 to 450 nm.

9. The endoscope apparatus according to claim 11, wherein the second irradiation windows generates (i) narrow bandwidth of light having a wavelength in which a light absorption degree of reduced hemoglobin in blood is substantially equal to a light absorption degree of oxidized hemoglobin and (ii) narrow bandwidth of light having a wavelength in which both of the light absorption degrees have difference.

10. The endoscope apparatus according to any one of claims 7 to 9, wherein the second light source generates that the central wavelength of the narrow bandwidth of light is 620 to 680 nm.

11. The endoscope apparatus according to any one of claims 7 to 10, wherein the second light source generates that the central wavelength of the narrow bandwidth of light is 750 to 850 nm.

12. The endoscope apparatus according to any one of claims 7 to 10, wherein at least one the first and second light sources is made of semiconductor emitting element.

13. The endoscope apparatus according to any one of claims 1 to 12, further comprising:
an imaging element which detects image of the test object to be focused through the observation window and outputs observation view signal of the test object.

14. The endoscope apparatus according to any one of claims 1 to 13, wherein the plurality of irradiation windows and the observation window are disposed on a front end of the insertion unit to be inserted into a body cavity, and
the light sources supplies the light to at least one of the plurality of irradiation windows via at least one leading light units.
